# EUROPEAN PATENT SPECIFICATION

(11) **EP 3 886 794 B1**
(45) Date of publication and mention of the grant of the patent: **17.06.2026**
(21) Application number: 19805993.3
(22) Date of filing: 26.11.2019
(51) Int. Cl.: A61K 8/31, A61K 8/37, A61Q 19/00, A61K 8/88

(54) **COSMETIC COMPOSITION COMPRISING SPECIFIC HYPERBRANCHED COPOLYMERS AND OILS**
KOSMETISCHE ZUSAMMENSETZUNG MIT SPEZIFISCHEN HYPERVERZWEIGTEN COPOLYMEREN UND ÖLEN
COMPOSITION COSMÉTIQUE COMPRENANT DES COPOLYMÈRES HYPER RAMIFIÉS ET DES HUILES SPÉCIFIQUES

(30) Priority: 26.11.2018 EP 18208231
(43) Date of publication of application: 06.10.2021
(73) Proprietor: DSM IP Assets B.V., 6221 BE Maastricht (NL)
(72) Inventor: MENDROK-EDINGER, Christine, 4303 Kaiseraugst (CH)
(74) Representative: dsm-firmenich IP
(86) International application number: PCT/EP2019/082489
(87) International publication number: WO 2020/109262

(56) References cited:
- WO-A1-2006/013200
- WO-A1-2014/040811
- WO-A1-2014/041019
- WO-A1-2017/174214
- WO-A2-2007/144189
- "Research disclosure", RESEARCH DISCLOSURE, KENNETH MASON PUBLICATIONS, HAMPSHIRE, UK, GB, vol. 606, no. 33, 1 October 2014 (2014-10-01), pages 5, XP007143477, ISSN: 0374-4353, [retrieved on 20140916]

## Description

### Technical Field

The present invention relates to the field of cosmetics, particularly to field of skin care and sun care.

### Background of the invention

In the field of cosmetic the details of a cosmetic composition, i.e. a formulation, are very sensitive to aspects of texture, feel and visual aspects.

A big problem is that material of such a composition when applied to a surface, particularly to the skin, can be transferred to another surface when said other surface is brought in contact with said composition. Such a transfer is negative in two ways: First of all, the transfer of the material is not desired as it is removed from the site of action, such as treating, moisturizing or protecting the skin. Secondly, the surface of contact is contaminated with said material. Particularly fabrics, such as clothes can be stained. Furthermore, decorative or functional surfaces can be contaminated which is negative for the functionality, respectively, for their visual or aesthetic properties. Particularly, displays of mobile phones, screens, or touch screens can be negatively affected by such material transfer.

In a Research Disclosure published by Kenneth Mason Publications, Hampshire, UK, vol. 606, no. 33, 1 October 2014 (2014-10-01), page 5 onwards, an anti-dandruff shampoo was described comprising Polyquaternium- 1 10 and Hydrogenated Polydecene. In addition an Extra Shine Revitalizing Hair Cream was described comprising Polyquaternium- 1 10 jojoba seed oil and apricot kernel oil.

WO 2014/040811 A1 and WO 2014/041019 A1 describe hair care compositions comprising specific quaternized hyperbranched polymers having end-groups of a formula (I) characterized in that said quaternized hyperbranched polymer is obtainable by preparation of a hyperbranched polymer having dimethylamino end groups by condensation of 2-dodecen-1-ylsuccinic anhydride, diisopropanolamine and N,N-bis[3-(dimethylamino)propyl]amine followed by quaternization of the dimethylamino end-groups to end groups of the formula (I).

WO 2006/013200 A1 describes a cosmetic care and/or makeup composition comprising, in a cosmetically acceptable medium: - an organic liquid phase; and - at least one linear or branched polyesteramide polymer comprising in its main chain and/or in the form of one or more side grafts on its main chain: at least one aliphatic polyester group consisting of from 2 to 1000 aliphatic ester units, and at least two groups chosen from amide and/or sulfonamide groups

WO 2017/174214 A1 describes a composition comprising: (a) a sunscreen agent, (b) a hyperbranched or dendrimer hydrophilic polymer, (c) a film forming polymer different from (b), and wherein the composition comprises a polar solvent present at up to 95 wt % of the composition.

WO 2007/144189 A2 describes personal care, home care and fabric care compositions based on hyperbranched condensation polymers having in the polymer backbone at least one unit comprising an amide bond attached to a trismethylene-aminomethane group and to novel hyperbranched condensation polymers.

### Summary of the invention

Hence, there exist a great desire to reduce such a transfer of cosmetic compositions from surface to surface when the surfaces are contacted.

Surprisingly, it has been found that the cosmetic compositions of claim 1 can solve this problem.

It has been found that the combination of a specific hyperbranched copolymer and oil(s) of a specific polarity index heavily reduce the material transfer of such compositions to contacted surfaces.

Further aspects of the invention are subject of further independent claims. Particularly preferred embodiments are subject of dependent claims.

### Detailed description of the invention

In a first aspect the present invention relates to a cosmetic composition comprising a hyperbranched copolymer of the monomers
(i) dodecenyl succinic acid anhydride
(ii) diisopropanol amine
(iii) bis-dimethylaminopropyl amine

having terminal groups of formula 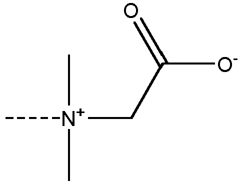
and having a molecular weight Mn of between 1200 and 4000 g/mol;
and at least one oil with a polarity index of in the range of 18-5 mN/m;
whereas the polarity index of the oil is the interfacial tension of the oil with respect to water and is determined using a ring tensiometer in analogy to the ASTM method D971-99a (2004),
characterized in that the oil is di-C12-13 alkyl tartrate.

In a further aspect the present invention relates to a use of a hyperbranched copolymer of the monomers
(i) dodecenyl succinic acid anhydride
(ii) diisopropanol amine
(iii) bis-dimethylaminopropyl amine

having terminal groups of formula 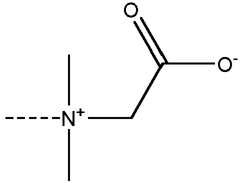
and having a molecular weight Mn of between 1200 and 4000 g/mol

in a cosmetic composition comprising at least one oil with a polarity index of either in the range of 50-30 mN/m or in the range of 18-5 mN/m for reducing the material transfer to a contacted surface;
whereas the polarity index of the oil is the interfacial tension of the oil with respect to water and is determined using a ring tensiometer in analogy to the ASTM method D971-99a (2004).

The term "molecular weight Mn" stands for the number average molecular weight.

The term "oil" in the present description stands for a water-insoluble, organic, natural, or synthetic, cosmetically suitable oil which preferably has a liquid or viscous consistency at 23° C. "Water-insoluble" in this context refers to a water-solubility of the oil of not more than 2 % by weight at 20° C.

The term "polarity index" is a parameter which is known to the person skilled in the art. The polarity of an oil is defined as the polarity index (interfacial tension) of the oil with respect to water. The interfacial tension, i. e. the polarity index, can be particularly determined using a ring tensiometer (e.g., Krüss K 10), which measures the interfacial tension in mN/m in analogy to the ASTM method D971-99a (2004).

The term "preparation" or "formulation" is used in this document as equivalent to the term "composition".

The term "material transfer" in this document means the mass transfer of the cosmetic composition or some ingredients thereof when the cosmetic composition is applied to a surface and afterwards said surface is brought in contact with a surface of a different object and separated again. By this contact some material is transferred from the first surface to the surface of the different object. The amount of material transferred can be determined by measuring the weight gain of the second object.

The hyperbranched copolymer is preferably prepared by the following consecutive steps of:
a1) polymerizing the monomers (i) and monomers (ii) and monomers (iii) to yield a polyesteramide having terminal dimethyl amino groups of the formula\
a2) quaternization of the dimethyl amino groups of the polyesteramide of step a1) by 2-chloroacetate, particularly by sodium 2-chloroacetate.

Details for the polymerization step a1) to yield the respective polyesteramide having terminal dimethyl amino groups of the formula 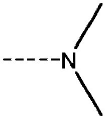 are disclosed for example by EP 2 794 729 B1.

Preferably in the polymerization step a1) the monomer (iii) is added to a mixture of monomers (ii) and (iii) under stirring, followed by heating.

Details of the quaternization step a2) are disclosed as well by EP 2 794 729 B1.

The amount of the hyperbranched copolymer of the monomers
(i) dodecenyl succinic acid anhydride
(ii) diisopropanol amine
(iii) bis-dimethylaminopropyl amine

having terminal groups of formula 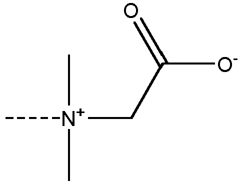
and having a molecular weight Mn of between 1200 and 4000 g/mol
in the cosmetic composition is typically between 0.1 and 3 % by weight, based on the total weight of the cosmetic composition.

It is preferred that the molar ratio of the monomers (i) to monomers (ii) is between 5: 1 and 0.5 : 1, particularly between 4 : 1 and 1 : 1, preferably between 3 : 1 and 3 : 2.

It is further preferred that the molar ratio of the monomers (i) to monomers (iii) is between 5 : 1 and 0.5 : 1, particularly between 3 : 1 and 1 : 1, preferably between 2.5 : 1 and 1.1 : 1.

The hyperbranched copolymer has preferably a number average molecular weight Mₙ of between 1400 and 3000 g/mol, preferably between 2100 and 2300 g/mol.

Preferably, the hyperbranched polymer is polyquaternium-110, also identified by CAS Number 1323977-82-7.

The cosmetic composition further comprises at least one oil with a polarity index in the range of 18-5 mN/m, characterized in that the oil is di-C12-13 alkyl tartrate.

The oils in the above defined use are particularly oils of the chemical classes of alkanes, triglycerides, ester oils or glycol esters.

Particularly, the oil is an oil which is selected from the group consisting of the oils listed in table 1.

**Table 1. suitable oils**

| **Oil** | **PI¹** | **Oil** | **PI¹** |
|---|---|---|---|
| Cycloparaffin | 49.1 | Dicaprylyl Carbonate | 31.7 |
| Polydecene | 46.7 | Octyl Isostearate | 31.6 |
| Squalane | 46.2 | Trimethylhexyl Isononanoate | 31.1 |
| Hydrogenated Polyisobutene | 44.7 | 2-Ethylhexyl Isononanoate | 31 |
| Isohexadecane | 43.8 | Dicaprylyl Ether | 30.9 |
| Paraffin oil perliquidum | 43.7 | Dihexyl Carbonate | 30.9 |
| Polydimethylsiloxane | 42.4 | Octyl Cocoate | 30 |
| Isoeicosane | 41.9 | Olive oil | 16.9 |
| Ethoxydiglycol Oleate | 40.5 | Dibutyl adipate | 14.3 |
| Decyl Olivate | 40.3 | Castor oil | 13.7 |
| Dioctylcyclohexane | 39 | Calendula oil | 11.1 |
| Paraffin oil subliquidum | 38.3 | Wheatgerm oil | 8.3 |
| Paraffinum Liquidum | 37.6 | Di-C12-13 Alkyl Tartrate | 7.1 |
| Isocetyl Palmitate | 36.2 | Propylene glycol monoisostearate | 6.2 |
| Cyclopentasiloxane | 32.3 | Cocoglycerides | 5.1 |

| | | | |
|---|---|---|---|
| ¹ PI = Polarity Index [mN/m] | | | |

Particularly suitable oils are hydrocarbon-based oils such as aliphatic and/or aromatic oils which have preferably 8 to 32, more particularly 15 to 20, carbon atoms. Examples are squalane, liquid paraffins, isohexadecane, isoeicodecane, polyolefins such as polydecenes, hydrogenated polyisobutenes, dialkylcyclohexane, and mineral oil. Particularly suitable are liquid paraffins such as the low-viscosity paraffins (paraffinum perliquidum), which have a viscosity of 25 to 80 mPa·s, and/or the high-viscosity paraffins (paraffinum subliquidum), which as oily liquids have a viscosity of 110 to 230 mPa·s. Hydrogenated polyisobutenes are available, for example, under the trade name Luvitol^{®} Lite from BASF SE.

In one preferred embodiment, the cosmetic composition comprises at least one oil with a polarity index of between 15 and 5 mN/m.

Preferably the oil is selected from the group consisting of dibutyl adipate, castor oil, calendula oil, wheatgerm oil, di-C12-13 alkyl tartrate, propylene glycol monoisostearate and cocoglycerides, particularly from the group consisting of di-C12-13 alkyl tartrate, dibutyl adipate and cocoglycerides.

Preferably the oil with a polarity index of between 50 and 30 mN/m is selected from the group consisting of isoparaffin (C12-C14), cycloparaffin, polydecene, squalane, hydrogenated polyisobutene, isohexadecane, paraffin oil perliquidum, polydimethylsiloxane, isoeicosane, ethoxydiglycol oleate, decyl olivate, dioctylcyclohexane, paraffin oil subliquidum, paraffinum liquidum, isocetyl palmitate, cyclopentasiloxane, dicaprylyl carbonate, octyl isostearate, trimethylhexyl isononanoate, 2-ethylhexyl isononanoate, dicaprylyl ether, dihexyl carbonate and octyl cocoate, particularly from the group consisting of di-caprylyl carbonate, paraffinum liquidum, and squalane.

The oils having a polarity index the range 18-5 mN/m, particularly in the range of 15-5 N/m, are preferred over the oils having a polarity index in the range of 50-30. The most preferred oils are di-C12-13 alkyl tartrate and dibutyl adipate.

The total amount of the oil(s) with a polarity index of either in the range of 50 - 30 mN/m or in the range of 18 - 5 mN/m is preferably in the range between 2 - 20 % by weight, particularly 3 - 15 % by weight, based on the total weight of the cosmetic composition.

The cosmetic composition further preferably comprises at least one emulsifier, preferably an anionic emulsifier. Preferably the anionic emulsifier is an anionic emulsifier selected from the group consisting of potassium cetyl phosphate, disodium cetearyl sulfosuccinate, sodium stearoyl glutamate, sodium stearoyl lactylate, glyceryl stearate citrate and sodium cocoyl isethionate.

Potassium cetyl phosphate is commercially available as Amphisol^{®} K at DSM Nutritional Products Ltd Kaiseraugst.

The amount of emulsifier is preferably in the range between 0.1 - 6.0 % by weight, more preferably between 0.25 - 5.0 % by weight, particularly between 0.5 - 4.0 % by weight, based on the total weight of the cosmetic composition.

The composition is preferably sulfate-free.

Hence, the cosmetic composition is preferably particularly free of sulfates of the group consisting of alkyl sulfates, alkyl ether sulfates, alkyl amido ether sulfates, alkylaryl polyether sulfates and monoglycerides sulfate as well as mixtures thereof.

The term "free" as used in the present document, for example in "sulfate-free", is used to mean that the respective substance is only present at amounts of less than 0.5 % by weight, particularly less than 0.1 % by weight, more particularly below 0.05 % by weight, relative to the weight of the composition. Preferably, "free" means that the respective substance is completely absent in the composition.

The term "sulfate-free" is used in the present document to mean that the composition is free of any anionic tenside having a terminal anionic group of the formula

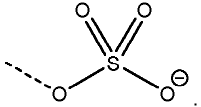

The cosmetic composition is preferably free of cationic emulsifiers. Typical example for such cationic emulsifiers are isostearamidopropyl dimethylamine, stearalkonium chloride, stearamidoethyl diethylamine, behentrimonium methosulfate, behenoyl PG-trimonium chloride, cetrimonium bromide, behenamidopropyl dimethylamine behenate, brassicamidopropyl dimethylamine, stearamidopropyl dimethylamine stearate, cocamidopropyl PG-dimonium chloride, distearoylethyl hydroxyethylmonium methosulfate, dicocoylethyl hydroxyethylmonium methosulfate, distearoylethyl dimonium chloride, shea butteramidopropyltrimonium chloride, behenamidopropyl dimethylamine, brassicyl isoleucinate esylate, acrylamidopropyltrimonium chloride/acrylates copolymer, linoleamidopropyl ethyldimonium ethosulfate, dimethyl lauramine isostearate, isostearamidopropyl laurylacetodimonium chloride, particularly behentrimonium chloride, distearyldimonium chloride, cetrimonium chloride, steartrimonium chloride, and palmitamido-propyltrimonium chloride.

The cosmetic composition further may comprise cosmetic carriers, excipients and diluents as well as additives and active ingredients commonly used in the skin care industry which are suitable for use in the cosmetic compositions of the present invention are for example described in the International Cosmetic Ingredient Dictionary & Handbook by Personal Care Product Council (http://www.personalcarecouncil.org/), accessible by the online INFO BASE (http://online.personalcarecouncil.org/jsp/Home.jsp), without being limited thereto.

Such possible ingredients of the cosmetic composition are particularly enhance the performance and/or consumer acceptability such as preservatives, antioxidants, fatty substances/oils, thickeners, softeners, light-screening agents, moisturizers, fragrances, co-surfactants, fillers, sequestering agents, cationic-, nonionic- or amphoteric polymers or mixtures thereof, acidifying or basifying agents, viscosity modifiers, and natural hair nutrients such as botanicals, fruit extracts, sugar derivatives and/or amino acids or any other ingredients usually formulated into cosmetic compositions. The necessary amounts of the adjuvants and additives can, based on the desired product, easily be chosen by a person skilled in the art in this field and will be illustrated in the examples, without being limited hereto.

The additional ingredients can either be added to the oily phase, the aqueous phase or separately as deemed appropriate.

In an advantageous embodiment, the compositions according to the present invention comprise from 50% to 99%, preferably from 60% to 98%, more preferably from 70% to 98%, such as in particular from 80% to 95% of a carrier, based on the total weight of the cosmetic composition.

In a particular advantageous embodiment, the carrier consists furthermore of at least 40 wt. %, more preferably of at least 50 wt.-%, most preferably of at least 55 wt.-% of water, such as in particular of 55 to 90 wt.-% of water.

Furthermore, it is preferred that the cosmetic composition comprises at least one UV filter. The UV filter may be a liquid or solid UV filter. The UV filter may be a UV-A or UV-B filters.

Suitable liquid UV-filter absorb light in the UVB (280 - 315 nm) and/ or UVA (315 - 400 nm) range and are liquid at ambient temperature (i.e. 25°C). Such liquid UV-filter are well known to a person in the art and encompass in particular cinnamates such as e.g. octyl methoxycinnamate (PARSOL^{®} MCX) and isoamyl methoxycinnamate (Neo Heliopan^{®} E 1000), salicylates such as e.g. homosalate (3,3,5 trimethylcyclohexyl 2-hydroxybenzoate, PARSOL^{®} HMS) and ethylhexyl salicylate (also known as ethylhexyl salicylate, 2 ethylhexyl 2-hydroxybenzoate, PARSOL^{®} EHS), acrylates such as e.g. octocrylene (2 ethylhexyl 2-cyano-3,3-diphenylacrylate, PARSOL^{®} 340) and ethyl 2-cyano-3,3 diphenylacrylate, esters of benzalmalonic acid such as in particular dialkyl benzalmalonates such as e.g. di (2-ethylhexyl) 4-methoxybenzalmalonate and polysilicone 15 (PARSOL^{®} SLX), dialkylester of naphthalates such as e.g. diethylhexyl 2,6-naphthalate (Corapan^{®} TQ), syringylidene malo-nates such as e.g. diethylhexyl syringylidene malonate (Oxynex^{®} ST liquid) as well as benzotriazolyl dodecyl p-cresol (Tinoguard^{®} TL) as well as benzophenone-3 and drometrizole trisiloxane.

Particular advantageous liquid UV-filter are octyl methoxycinnamate, homosalate, ethylhexyl salicylate, octocrylene, diethylhexyl 2,6-naphthalate, diethylhexyl syringylidene malonate, benzotriazolyl dodecyl p-cresol, benzophenone-3, drometrizole trisiloxane, Polysilicone-15 as well as mixtures thereof.

Suitable solid UV-filter absorb light in the UVB and/ or UVA range and are solid at ambient temperature (i.e. 25°C). They are particularly solid organic UV filters. Particularly suited solid UV-filters are of the group consisting of bis-ethylhexyloxyphenol methoxyphenyl triazine, butyl methoxydibenzoyl methane, methylene bis-benzotriazolyl tetramethylbutylphenol, diethylamino hydroxybenzoyl hexyl benzoate, ethylhexyl triazone, diethylhexyl butamido triazone, 4-methylbenzylidene camphor and 1,4-di(benzoxazol-2'-yl)benzene.

The amount of an individual organic UV filter is preferably in the range of 0.1 to about 6 % by weight, preferable in the range of 0.5 to 5 % by weight, most preferably in the range of 1 to 4 % by weight, based on the total weight of the cosmetic composition.

In case of a sun care composition, the total amount of organic UV filter (s) depends strongly on the targeted UV protection of said composition and is typically in the range of between 1 to 50% by weight, preferably between 5 to 40% by weight, based on the total weight of said composition.

A sun creme with an SPF 15 (SPF=sun protection factor), for example, comprises preferably a total amount of organic UV filter (s) of between 4 to 20% by weight, more preferably between 7 and 15 % by weight, based on the total weight of said composition.

A sun creme with an SPF 30, for example, comprises preferably a total amount of organic UV filter (s) of between 10 to 40% by weight, more preferably between 15 and 25 % by weight, based on the total weight of said composition.

A sun creme with an SPF 50, for example, comprises preferably a total amount of organic UV filter (s) of between 15 to 50% by weight, more preferably between 20 and 40 % by weight, based on the total weight of said composition.

Particularly suitable thickeners in all embodiments of the present invention are xanthan gum, gellan gum and/ or carboxymethylcellulose. Most preferably in all embodiments of the present invention the thickener is xanthan gum or gellan gum.

Such thickener(s) are preferably used in an amount (total) selected in the range from 0.1 to 1 wt.-%, more preferably in an amount of 0.1 to 0.5 wt.-%, based on the total weight of the cosmetic composition.

It is preferred that the composition is free of polyvinylpyrrolidones (PVP), particularly free of alkylated polyvinylpyrrolidiones, such as copolymers of N-vinylpyrrolidones and hexadecane or eicosene, e.g. as commercially available as Antaron V-216 or Antaron V-220.

The cosmetic compositions according to the invention in general have a pH in the range from 3 to 10, preferably a pH in the range from 4 to 8 and most preferably a pH in the range from 4 to 7.5. The pH can easily be adjusted as desired with suitable acids such as e.g. citric acid or bases such as NaOH according to standard methods in the art.

The cosmetic composition is preferably sulfate-free and/or free of parabens, and/or silicon oils and/or silicone surfactants and/or methylisothiazolidine and/or free of polyvinylpyrrolidones (PVP), particularly free of alkylated polyvinylpyrrolidiones.

The cosmetic composition is preferably a topical composition.

The term "topical" as used herein is understood here to mean external application to keratinous substances, which are in particular the skin, scalp, eyelashes, eyebrows, nails, mucous membranes and hair, preferably the skin.

As the topical compositions are intended for topical application, it is well understood that they comprise a physiologically acceptable medium, i.e. a medium compatible with keratinous substances, such as the skin, mucous membranes, and keratinous fibres. In particular, the physiologically acceptable medium is a cosmetically acceptable carrier.

The term "cosmetically acceptable carrier" refers to all carriers and/or excipients and/ or diluents conventionally used in cosmetic compositions such as in particular in sun care products.

Preferably the cosmetic composition is a skin care preparation, decorative preparation, or a functional preparation.

Examples of skin care preparations are, in particular, light protective preparations, anti-ageing preparations, preparations for the treatment of photo-ageing, body oils, body lotions, body gels, treatment creams, skin protection ointments, skin powders, moisturizing gels, moisturizing sprays, face and/or body moisturizers, skin-tanning preparations (i.e. compositions for the artificial/sunless tanning and/or browning of human skin), for example self-tanning creams as well as skin lightening preparations.

Examples of functional preparations are cosmetic or pharmaceutical compositions containing active ingredients such as hormone preparations, vitamin preparations, vegetable extract preparations and/or anti-ageing preparations without being limited thereto.

The cosmetic composition is preferably a skin care composition.

In a particular embodiment, the cosmetic composition is a sun care composition. Sun care compositions are light-protective preparations (sun care products), such as sun protection milks, sun protection lotions, sun protection creams, sun protection oils, sun blocks or day care creams with a SPF (sun protection factor). Of particular interest are sun protection creams, sun protection lotions, sun protection milks and sun protection preparations.

The cosmetic compositions according to the present invention may be in the form of a suspension or dispersion in solvents or fatty substances, or alternatively in the form of an emulsion or micro emulsion (in particular of oil-in-water (O/W-) or water-in-oil (W/O-)type, silicone-in-water (Si/W-) or water-in-silicone (W/Si-)type, PIT-emulsion, multiple emulsion (e.g. oil-in-water-in oil (O/W/O-) or water-in-oil-in-water (W/O/W-)type), pickering emulsion, hydrogel, alcoholic gel, lipogel, one- or multiphase solution or vesicular dispersion or other usual forms, which can also be applied by pens, as masks or as sprays.

Preferred cosmetic compositions in all embodiments of the present invention are emulsions which contain an oily phase and an aqueous phase such as in particular O/W, W/O, Si/W, W/Si, O/W/O, W/O/W multiple or a pickering emulsions.

The total amount of the oily phase (i.e. the phase containing all oils and fats including the oils with a polarity index of either in the range of 50-30 mN/m or in the range of 18-5 mN/m) present in such emulsions is preferably at least 10 wt.-%, such as in the range from 10 to 60 wt.-%, preferably in the range from 15 to 50 wt.-%, most preferably in the range from 15 to 40 wt.-%, based on the total weight of the cosmetic composition.

The amount of the aqueous phase present in such emulsions is preferably at least 20 wt. %, such as in the range from 40 to 90 wt.-%, preferably in the range from 50 to 85 wt.-%, most preferably in the range from 60 to 85 wt.-%, based on the total weight of the cosmetic composition.

More preferably, the cosmetic compositions according to the present invention are in the form of an oil-in-water (O/W) emulsion comprising an oily phase dispersed in an aqueous phase in the presence of an O/W- respectively Si/W-emulsifier as such compositions show a significantly pronounced reduction of the transfer of the respective composition to surfaces. The preparation of such O/W emulsions is well known to a person skilled in the art.

The compositions in form of O/W emulsions according to the invention can be provided, for example, in all the formulation forms for O/W emulsions, for example in the form of serum, milk or cream, and they are prepared according to the usual methods. The compositions which are subject-matters of the invention are preferably intended for topical application and can in particular constitute a dermatological or cosmetic composition, for example intended for protecting human skin against the adverse effects of UV radiation (antiwrinkle, anti-ageing, moisturizing, anti-sun protection and the like).

It has been found that the cosmetic compositions have a strongly reduced material transfer to a contacted surface when the specific hyperbranched copolymer of the monomers
(i) dodecenyl succinic acid anhydride
(ii) diisopropanol amine
(iii) bis-dimethylaminopropyl amine

having terminal groups of formula 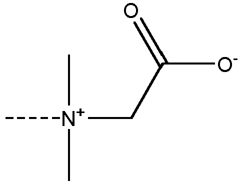
having a molecular weight Mn of between 1200 and 4000 g/mol is used together with an oil having a polarity index of either in the range of 50-30 mN/m or in the range of 18-5 mN/m in a cosmetic composition.

Hence, a further aspect of the present invention is the use of a hyperbranched copolymer of the monomers
(i) dodecenyl succinic acid anhydride
(ii) diisopropanol amine
(iii) bis-dimethylaminopropyl amine

having terminal groups of formula 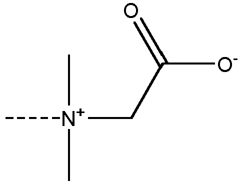
and having a molecular weight Mn of between 1200 and 4000 g/mol
in a cosmetic composition comprising at least one oil with a polarity index of either in the range of 50-30 mN/m or in the range of 18-5 mN/m for reducing the material transfer to a contacted surface.

The amount of material transfer is determined by determination of the weight of the object (second object) before and after contact. Any weight gain after contact is due to a material transfer from the first to the second object. The reduction of material transfer is determined by comparing compositions according to the inventions with the respective (not according to the invention) composition which does not contain the above hyperbranched copolymer, respectively the oil. The reduction is expressed in % of the material transfer of the two measurements.

It has been found that the reduction of more than 50 %, even more than 60%, particularly more than 70 %, can be obtained.

The cosmetic composition is applied to a first surface. Said surface is preferably skin, particularly human skin. It has been found that using a porous sponge instead of skin is a good approach for simulate a material transfer from skin to another surface.

The surface of the contacted object (second object) is preferably a glass surface or a plastic or a surface of a fabric.

In case the surface is a fabric, this is very advantages to avoid an unwanted transfer of cosmetic composition to a fabric, particularly to clothes, as the cosmetic composition might stain the fabric.

Particularly, the contacted surface (i.e. surface of second object) is a glass surface.

Most preferably, the contacted surface (i.e. surface of second object) is an optical glass such as used for reading glasses or sunglasses or a display of screen of a smartphone display of a mobile phone, computer device or tablet.

By reducing the material transfer of the cosmetic composition, particularly the problem of marks, particularly finger marks, left on glasses such as optical glasses of instruments or visual glasses when said glass surface is contacted with fingers can be reduced or even avoided. Particularly, this can heavily reduce or even avoid any undesired effects on the light rays transmitted through said glass by said material left on the surface.

Furthermore, marks left on the surface of an aesthetic surface such as of a mirror or a highly glossy or highly mat surface such as a of a top coat of a car or furniture or piece of art, can be strongly reduced. This is very advantageous as such surfaces need a high amount of cleaning maintenance, if they are brought in contact with skin on which cosmetic compositions have been applied, particularly if they are touched by fingers which have been previously in contact with cosmetic compositions.

Marks left on the surface of display units, such as displays of mobile phones, screens, or touch screens of monitors, laptops, mobile phones or tablets can be strongly reduced. As a result of this, the readability can be improved. As the functionality of touch screens depends on surface aspects, the invention helps also to improve constant touch screen functionality without excessive need of cleaning said glass surface.

The reduction of material transfer also heavily reduces the labour and cost involved in the cleaning of said surfaces when they are contacted with skin.

### Examples

The present invention is further illustrated by the following experiments. These examples are illustrative only and are not intended to limit the scope of the invention in any way.

### Preparation of hyperbranched copolymer (HBC1)

The hyperbranched copolymer *HBC1* of the monomers dodecenyl succinic acid anhydride and diisopropanol amine and bis-dimethylaminopropyl amine has been prepared according to example 3 in EP 2 794 729 B1 using 237.59 g of N,N-bis(N'N'-dimethylaminopropyl)amine and 112.6 g diisopropanol amine and 426.89 g of dodecenylsuccinic anhydride. After heating and vacuum, the residual carboxylic acid content of < 0.3 meq/g (tritrimetrical analysis) AV=9.8mg KOH/g and amine content of 2.99 meq/g (tritrimetrical analysis) and a molecular weight Mn=2240 was obtained. This product has been reacted with sodium chloroacetate in water and stirred at 80°C until ¹H-NMR analysis shows a complete conversion of the chloroacetate to obtain the hyperbranched copolymer *HBC1* which has terminal groups of the formula 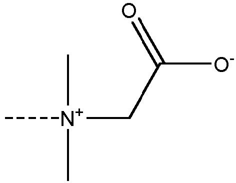 and a molecular weight Mn of 2.3 kDa.

The hyperbranched copolymer *HBC1* was used as a 45 % solution in water in the following experiments.

As a reference a hyperbranched copolymer Hybrane S1200 has been used. Hybrane S1200 is a hyperbranched copolymer but has a different structure as *HPC1,* respectively as requested for the present invention. In the table 4 Hybrane S1200 is abbreviated as *Ref-HBC* and used as a 45 % solution in water.

### Material Transfer

The material transfer has been determined with the sponge test as outlined in the following:
- Cut a sponge cloth (Weitawip Claire, from Weita AG: cellulose/cotton fiber mixture, 200 g/m², 5 mm thickness) into pieces of 76mm x 26mm
- Tare the sponge sample
- Apply 350 mg of the respective sample (= cosmetic composition) and distribute homogenously all over the sponge surface of 76 mm x 26 mm
- Weigh the sponge with the applied sample
- Tare a microscope slide (glass plate 76 mm x 26 mm x 1 mm)
- Put the microscope slide (glass plate) on top of the sponge, on which a balance weight of 500g (height: 6.3 cm, diameter at area of contact: 3.7 cm) is placed for 10 seconds to apply a specific pressure to the sample
- Remove cautiously vertically the microscope slide
- Weigh the removed microscope slide and determine accordingly the amount of sample transferred to the glass plate
- Repeat the test for each composition 10 times to receive an average value (mean value) for each sample.

### Preparation of cosmetic compositions

The ingredients (in % by weight) of the oil phase according to tables 2 to 4 have been combined and heated up to 85°C. The ingredients (in % by weight) of the water phase according to tables 2 to 4 have been combined, stirred until the Xanthan Gum is completely dissolved and then heated up to 80°C. The two phases have been combined and homogenized for 1 minute with 10'000 rpm. Under moderate stirring the emulsion is cooled down to 35°C. In case a hyperbranched copolymer is used in an example, the respective hyperbranched polymer (in % by weight) is now added to the emulsion under stirring. The stirring has been continued until the emulsion reached room temperature. The amounts of ingredients of the compositions (sum up to 100 % by weight) used are chosen so that each composition has a total weight of 70 grams.

Examples 1-8 are all in accordance with the use of the invention as defined in claim 11 but only the compositions of Examples 2 and 7 are in accordance with the composition of the invention as defined in claim 1.

**Table 2. Material transfer of cosmetic compositions and respective reductions.**

| Ingredient (INCI) | PI¹ [mN/m] | ***Ref.1*** | ***1*** | ***Ref.2*** | ***2*** | ***Ref.3*** | ***3*** |
|---|---|---|---|---|---|---|---|
| Oil Phase | | | | | | | |
| potassium cetyl phosphate | | 2.0 | 2.0 | 2.0 | 2.0 | 2.0 | 2.0 |
| ceteraryl alcohol | | 2.0 | 2.0 | 2.0 | 2.0 | 2.0 | 2.0 |
| stearyl alcohol | | 2.0 | 2.0 | 2.0 | 2.0 | 2.0 | 2.0 |
| cocoglycerides | 5.1 | 15.0 | 15.0 | | | | |
| di-C12-13 alkyl tartrate | 7.1 | | | 15.0 | 15.0 | | |
| dibutyl adipate | 14.3 | | | | | 15.0 | 15.0 |
| *C12-15 alkyl benzoate* | *21.8* | | | | | | |
| dicaprylyl carbonate | 31.7 | | | | | | |
| paraffinum liquidum | 37.6 | | | | | | |
| squalane | 46.2 | | | | | | |
| | | | | | | | |

| Water phase | | | | | | | |
|---|---|---|---|---|---|---|---|
| aqua | | Ad 100 | Ad 100 | Ad 100 | Ad 100 | Ad 100 | Ad 100 |
| glycerin | | 3.0 | 3.0 | 3.0 | 3.0 | 3.0 | 3.0 |
| xanthan gum | | 0.2 | 0.2 | 0.2 | 0.2 | 0.2 | 0.2 |
| Preservative (phenoxyethanol) | | 1.0 | 1.0 | 1.0 | 1.0 | 1.0 | 1.0 |
| | | | | | | | |
| ***HBC1*** (45% aq. solution) | | | 6.0 | | 6.0 | | 6.0 |
| Transfer [%]² | | 1.76 | 0.88 | 3.05 | 0.63 | 2.71 | 1.08 |
| Reduction [%]³ | | | 50.00 | | 79.34 | | 60.15 |

| | | | | | | | |
|---|---|---|---|---|---|---|---|
| ¹ PI = Polarity Index ² Material transfer of cosmetic composition in respect of composition applied to the sponge ³ Reduction of material transfer as compared to the respective reference (e.g. for 1: 50.00 % = 100% - 100*(0.88/1.76)). | | | | | | | |

**Table 2 (cont.) Material transfer of cosmetic compositions and respective reductions.**

| Ingredient (INCI) | PI¹ [mN/m] | ***Ref.4*** | ***4*** | ***Ref.5*** | ***5*** | ***Ref. 6*** | ***6*** | ***Ref. 7*** | ***Ref.8*** |
|---|---|---|---|---|---|---|---|---|---|
| Oil Phase | | | | | | | | | |
| potassium cetyl phosphate | | 2.0 | 2.0 | 2.0 | 2.0 | 2.0 | 2.0 | 2.0 | 2.0 |
| ceteraryl alcohol | | 2.0 | 2.0 | 2.0 | 2.0 | 2.0 | 2.0 | 2.0 | 2.0 |
| stearyl alcohol | | 2.0 | 2.0 | 2.0 | 2.0 | 2.0 | 2.0 | 2.0 | 2.0 |
| cocoglycerides | 5.1 | | | | | | | | |
| di-C12-13 alkyl tartrate | 7.1 | | | | | | | | |
| dibutyl adipate | 14.3 | | | | | | | | |
| *C12-15 alkyl benzoate* | *21.8* | | | | | | | *15.0* | *15.0* |
| dicaprylyl carbonate | 31.7 | 15.0 | 15.0 | | | | | | |
| paraffinum liquidum | 37.6 | | | 15.0 | 15.0 | | | | |
| squalane | 46.2 | | | | | 15.0 | 15.0 | | |
| | | | | | | | | | |

| Water phase | | | | | | | | | |
|---|---|---|---|---|---|---|---|---|---|
| aqua | | Ad 100 | Ad 100 | Ad 100 | Ad 100 | Ad 100 | Ad 100 | Ad 100 | Ad 100 |
| glycerin | | 3.0 | 3.0 | 3.0 | 3.0 | 3.0 | 3.0 | 3.0 | 3.0 |
| xanthan gum | | 0.2 | 0.2 | 0.2 | 0.2 | 0.2 | 0.2 | 0.2 | 0.2 |
| Preservative (phenoxyethanol) | | 1.0 | 1.0 | 1.0 | 1.0 | 1.0 | 1.0 | 1.0 | 1.0 |
| | | | | | | | | | |
| ***HBC1*** (45% aq. solution) | | | 6.0 | | 6.0 | | 6.0 | | 6.0 |
| Transfer [%]² | | 1.94 | 0.76 | 2.26 | 0.93 | 1.56 | 0.76 | 1.25 | 0.76 |
| Reduction [%]³ | | | 60.82 | | 58.85 | | 51.28 | | 39.20 |

| | | | | | | | | | |
|---|---|---|---|---|---|---|---|---|---|
| ¹ PI = Polarity Index ² Transfer of cosmetic composition in respect of composition applied to the sponge ³ Reduction of material transfer as compared to the respective reference | | | | | | | | | |

The results of table 2 clearly show that samples with the specific hyperbranched copolymer have a significant higher reduction in material transfer than the respective reference examples ("Ref.") without said copolymer. Furthermore, the results of table 2 also clearly show the influence of the polarity index of the oils used in the examples Ref.7 and Ref.8 as compared to the other examples 1-6 on the reduction of material transfer.

**Table 3. Material transfer of cosmetic compositions and respective reductions.**

| Ingredient (INCl) | PI¹ [mN/m] | ***Ref.2*** | ***2*** | ***7*** | ***Ref.4*** | ***4*** | ***8*** | ***Ref. 7*** | ***Ref.8*** | ***Ref.9*** |
|---|---|---|---|---|---|---|---|---|---|---|
| Oil Phase | | | | | | | | | | |
| potassium cetyl phosphate | | 2.0 | 2.0 | 2.0 | 2.0 | 2.0 | 2.0 | 2.0 | 2.0 | 2.0 |
| ceteraryl alcohol | | 2.0 | 2.0 | 2.0 | 2.0 | 2.0 | 2.0 | 2.0 | 2.0 | 2.0 |
| stearyl alcohol | | 2.0 | 2.0 | 2.0 | 2.0 | 2.0 | 2.0 | 2.0 | 2.0 | 2.0 |
| cocoglycerides | 5.1 | | | | | | | | | |
| di-C12-13 alkyl tartrate | 7.1 | 15.0 | 15.0 | 15.0 | | | | | | |
| *C12-15 alky benzoate* | *21.8* | | | | | | | *15.0* | *15.0* | *15.0* |
| dicaprylyl carbonate | 31.7 | | | | 15.0 | 15.0 | 15.0 | | | |
| | | | | | | | | | | |

| Water phase | | | | | | | | | | |
|---|---|---|---|---|---|---|---|---|---|---|
| aqua | | Ad 100 | Ad 100 | Ad 100 | Ad 100 | Ad 100 | Ad 100 | Ad 100 | Ad 100 | Ad 100 |
| glycerin | | 3.0 | 3.0 | 3.0 | 3.0 | 3.0 | 3.0 | 3.0 | 3.0 | 3.0 |
| xanthan gum | | 0.2 | 0.2 | 0.2 | 0.2 | 0.2 | 0.2 | 0.2 | 0.2 | 0.2 |
| Preservative (phenoxyethanol) | | 1.0 | 1.0 | 1.0 | 1.0 | 1.0 | 1.0 | 1.0 | 1.0 | 1.0 |
| Antaron V-216 | | | | 1.9 | | | 1.9 | | | 1.9 |
| | | | | | | | | | | |
| ***HBC1*** (45% aq. sol.) | | | 6.0 | 6.0 | | 6.0 | 6.0 | | 6.0 | 6.0 |
| Transfer [%]² | | 3.05 | 0.63 | 0.99 | 1.94 | 0.76 | 0.92 | 1.25 | 0.76 | 0.81 |
| Reduction [%]³ | | | 79.34 | 67.54 | | 60.82 | 52.58 | | 39.20 | 35.20 |
| Δ-Reduc.[%]⁴ | | | | 11.80 | | | 8.24 | | | 4.00 |

| | | | | | | | | | | |
|---|---|---|---|---|---|---|---|---|---|---|
| ¹ PI = Polarity Index ² Material transfer of cosmetic composition in respect of composition applied to the sponge ³ Reduction of material transfer as compared to the respective reference ⁴ Δ-Reduc.[%] = differences in the reduction (***2*** vs. ***7, 4*** vs. ***8, Ref.8*** vs ***Ref.9***). | | | | | | | | | | |

The results of table 3 show that samples comprising an alkylated polyvinylpyrrolidione, such as copolymers of N-vinylpyrrolidones and hexadecane (Antaron V-216) (***7, 8, Ref.9***) have a lower reduction of material transfer than the respective samples without alkylated polyvinylpyrrolidione (***2, 4, Ref.8***).

**Table 4. Material transfer of cosmetic compositions and respective reductions.**

| Ingredient (INCl) | PI¹ [mN/m] | ***Ref.4*** | ***4*** | ***Ref.10*** |
|---|---|---|---|---|
| Oil Phase | | | | |
| potassium cetyl phosphate | | 2.0 | 2.0 | 2.0 |
| ceteraryl alcohol | | 2.0 | 2.0 | 2.0 |
| ctearyl alcohol | | 2.0 | 2.0 | 2.0 |
| dicaprylyl carbonate | 31.7 | 15.0 | 15.0 | 15.0 |
| | | | | |

| Water phase | | | | |
|---|---|---|---|---|
| aqua | | Ad 100 | Ad 100 | Ad 100 |
| glycerin | | 3.0 | 3.0 | 3.0 |
| xanthan gum | | 0.2 | 0.2 | 0.2 |
| Preservative (phenoxyethanol) | | 1.0 | 1.0 | 1.0 |
| | | | | |
| ***HBC1*** (45% aq. sol.) | | | 6.0 | |
| ***Ref-HBC** (45% aq.sol.)* | | | | 6.0 |
| Transfer [%]² | | 1.94 | 0.76 | 0.84 |
| Reduction [%]³ | | | 60.82 | 56.70 |
| Δ-Reduc.[%]⁴ | | | | 4.12 |

| | | | | |
|---|---|---|---|---|
| ¹ PI = Polarity Index ² Material transfer of cosmetic composition in respect of composition applied to the sponge ³ Reduction of material transfer as compared to the respective reference ⁴ Δ-Reduc.[%] = differences in the reduction (***4*** vs. ***Ref.10***). | | | | |

The comparison of the example ***4*** with ***Ref.10*** in table 4 shows that the specific hyperbranched copolymer has a significant higher reduction in material transfer as when a hyperbranched copolymer with different structure is used.

The positive results and effect of reduction of material transfer by the composition according to the invention of the above experiments can also be found when the above compositions are applied to finger tips and a touch screen of an Samsung S9 display is touched with such a finger and the amounts of markings on the screen is visually assessed.

## Claims

1. A cosmetic composition comprising
a hyperbranched copolymer of the monomers
(i) dodecenyl succinic acid anhydride
(ii) diisopropanol amine
(iii) bis-dimethylaminopropyl amine
having terminal groups of formula 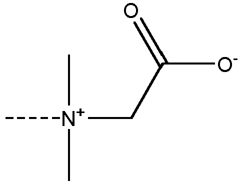
and having a molecular weight Mn of between 1200 and 4000 g/mol;
and at least one oil with a polarity index of in the range of 18-5 mN/m;
whereas the polarity index of the oil is the interfacial tension of the oil with respect to water and is determined using a ring tensiometer in analogy to the ASTM method D971-99a (2004),
**characterized in that** the oil is di-C12-13 alkyl tartrate.

2. The cosmetic composition according to claim 1 **characterized in that** the hyperbranched copolymer is prepared by the consecutive steps of
a1) polymerizing the monomers (i) and monomers (ii) and monomers (iii) to yield a polyesteramide having terminal dimethylamino groups of the formula 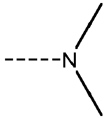
a2) quaternization of the dimethyl amino groups of the polyesteramide of step a1) by 2-chloroacetate, particularly by sodium 2-chloroacetate.

3. The cosmetic composition according to anyone of the preceding claims **characterized in that** the molar ratio of the monomers (i) to monomers (ii) is between 5 : 1 and 0.5 : 1, particularly between 4 : 1 and 1 : 1, preferably between 3 : 1 and 3 : 2.

4. **The** cosmetic composition according to anyone of the preceding claims **characterized in that** the molar ratio of the monomers (i) to monomers (iii) is between 5 : 1 and 0.5 : 1, particularly between 3 : 1 and 1 : 1, preferably between 2.5 : 1 and 1.1 : 1.

5. **The** cosmetic composition according to anyone of the preceding claims **characterized in that** the hyperbranched copolymer has a number average molecular weight Mₙ of between 1400 and 3000 g/mol, preferably between 2100 and 2300 g/mol.

6. **The** cosmetic composition according to anyone of the preceding claims **characterized in that** the hyperbranched copolymer is polyquaternium-110.

7. **The** cosmetic composition according to anyone of the preceding claims **characterized in that** the composition is free of polyvinylpyrrolidones (PVP).

8. **The** cosmetic composition according to anyone of the preceding claims, **characterized in that** the cosmetic composition is a skin care composition.

9. **The** cosmetic composition according to anyone of the preceding claims, **characterized in that** the cosmetic composition is a sulfate-free cosmetic composition.

10. **The** cosmetic composition according to anyone of the preceding claims, **characterized in that** the cosmetic composition is free of cationic emulsifier.

11. Use of a hyperbranched copolymer of the monomers
(i) dodecenyl succinic acid anhydride
(ii) diisopropanol amine
(iii) bis-dimethylaminopropyl amine
having terminal groups of formula 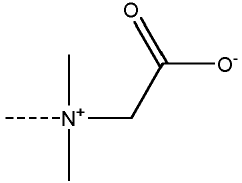
and having a molecular weight Mn of between 1200 and 4000 g/mol
in a cosmetic composition comprising at least one oil with a polarity index of either in the range of 50-30 mN/m or in the range of 18-5 mN/m for reducing the material transfer to a contacted surface;
whereas the polarity index of the oil is the interfacial tension of the oil with respect to water and is determined using a ring tensiometer in analogy to the ASTM method D971-99a (2004).

12. The use according to claim 11, **characterized in that** the contacted surface is a glass surface.

13. The use according to claim 11 or 12, **characterized in that** the contacted surface is a display of a mobile phone, computer device or tablet.

## Patentansprüche

1. Kosmetische Zusammensetzung, umfassend
ein hyperverzweigtes Copolymer der Monomere
(i) Dodecenylbernsteinsäureanhydrid
(ii) Diisopropanolamin
(iii) Bis(dimethylaminopropyl)amin
mit endständigen Gruppen der Formel 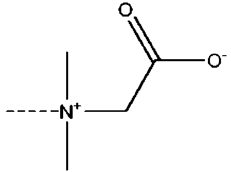
und mit einem Molekulargewicht Mn zwischen 1200 und 4000 g/mol;
und mindestens ein Öl mit einem Polaritätsindex im Bereich von 18-5 mN/m;
wobei es sich bei dem Polaritätsindex des Öls um die Grenzflächenspannung des Öls in Bezug auf Wasser handelt, die unter Verwendung eines Ringtensiometers in Analogie zur ASTM-Methode D971-99a (2004) bestimmt wird,
**dadurch gekennzeichnet, dass** es sich bei dem Öl um Di-C12-13-alkyltartrat handelt.

2. Kosmetische Zusammensetzung nach Anspruch 1, **dadurch gekennzeichnet, dass** das hyperverzweigte Copolymer hergestellt wird durch die aufeinanderfolgenden Schritte
a1) Polymerisieren der Monomere (i) und Monomere (ii) und Monomere (iii) unter Erhalt eines Polyesteramids mit endständigen Dimethylaminogruppen der Formel 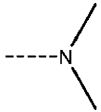
a2) Quaternisierung der Dimethylaminogruppen des Polyesteramids aus Schritt a1) mit 2-Chloracetat, besonders mit Natrium-2-chloracetat.

3. Kosmetische Zusammensetzung nach einem der vorhergehenden Ansprüche, **dadurch gekennzeichnet, dass** das Molverhältnis der Monomere (i) zu den Monomeren (ii) zwischen 5 : 1 und 0,5 : 1, besonders zwischen 4 : 1 und 1 : 1, bevorzugt zwischen 3 : 1 und 3 : 2 liegt.

4. Kosmetische Zusammensetzung nach einem der vorhergehenden Ansprüche, **dadurch gekennzeichnet, dass** das Molverhältnis der Monomere (i) zu den Monomeren (iii) zwischen 5 : 1 und 0,5 : 1, besonders zwischen 3 : 1 und 1 : 1, bevorzugt zwischen 2,5 : 1 und 1,1 : 1 liegt.

5. Kosmetische Zusammensetzung nach einem der vorhergehenden Ansprüche, **dadurch gekennzeichnet, dass** das hyperverzweigte Copolymer ein zahlenmittleres Molekulargewicht Mₙ zwischen 1400 und 3000 g/mol, bevorzugt zwischen 2100 und 2300 g/mol aufweist.

6. Kosmetische Zusammensetzung nach einem der vorhergehenden Ansprüche, **dadurch gekennzeichnet, dass** es sich bei dem hyperverzweigten Copolymer um Polyquaternium-110 handelt.

7. Kosmetische Zusammensetzung nach einem der vorhergehenden Ansprüche, **dadurch gekennzeichnet, dass** die Zusammensetzung frei von Polyvinylpyrrolidonen (PVP) ist.

8. Kosmetische Zusammensetzung nach einem der vorhergehenden Ansprüche, **dadurch gekennzeichnet, dass** es sich bei der kosmetischen Zusammensetzung um eine Hautpflegezusammensetzung handelt.

9. Kosmetische Zusammensetzung nach einem der vorhergehenden Ansprüche, **dadurch gekennzeichnet, dass** es sich bei der kosmetischen Zusammensetzung um eine sulfatfreie kosmetische Zusammensetzung handelt.

10. Kosmetische Zusammensetzung nach einem der vorhergehenden Ansprüche, **dadurch gekennzeichnet, dass** die kosmetische Zusammensetzung frei von kationischem Emulgator ist.

11. Verwendung eines hyperverzweigten Copolymers der Monomere
(i) Dodecenylbernsteinsäureanhydrid
(ii) Diisopropanolamin
(iii) Bis(dimethylaminopropyl)amin
mit endständigen Gruppen der Formel 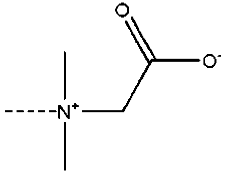
und mit einem Molekulargewicht Mn zwischen 1200 und 4000 g/mol
in einer kosmetischen Zusammensetzung, die mindestens ein Öl mit einem Polaritätsindex entweder im Bereich von 50-30 mN/m oder im Bereich von 18-5 mN/m umfasst, zur Verringerung der Stoffübertragung auf eine kontaktierte Oberfläche;
wobei es sich bei dem Polaritätsindex des Öls um die Grenzflächenspannung des Öls in Bezug auf Wasser handelt, die unter Verwendung eines Ringtensiometers in Analogie zur ASTM-Methode D971-99a (2004) bestimmt wird.

12. Verwendung nach Anspruch 11, **dadurch gekennzeichnet, dass** es sich bei der kontaktierten Oberfläche um eine Glasoberfläche handelt.

13. Verwendung nach Anspruch 11 oder 12, **dadurch gekennzeichnet, dass** es sich bei der kontaktierten Oberfläche um ein Display eines Mobiltelefons, Computergeräts oder Tablets handelt.

## Revendications

1. Composition cosmétique comprenant
un copolymère hyperramifié des monomères
(i) anhydride d'acide dodécénylsuccinique
(ii) diisopropanolamine
(iii) bis-diméthylaminopropylamine
ayant des groupes terminaux de formule 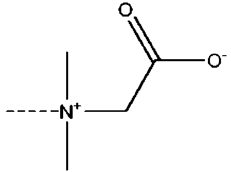
et ayant un poids moléculaire Mn compris entre 1 200 et 4 000 g/mole ;
et au moins une huile ayant un indice de polarité dans la plage de 18-5 mN/m ;
tandis que l'indice de polarité de l'huile est la tension interfaciale de l'huile vis-à-vis de l'eau et est déterminé au moyen d'un tensiomètre à anneau, par analogie avec le procédé de la norme ASTM D971-99a (2004),
**caractérisée en ce que** l'huile est un tartrate de di-C12-13 alkyle.

2. Composition cosmétique selon la revendication 1, **caractérisée en ce que** le copolymère hyperramifié est préparé par les étapes consécutives de
a1) polymérisation des monomères (i) et des monomères (ii) et des monomères (iii) pour donner un polyesteramide ayant des groupes diméthylamino terminaux de la formule 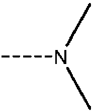
a2) quaternisation des groupes diméthylamino du polyesteramide de l'étape a1) par un 2-chloroacétate, en particulier par le 2-chloroacétate de sodium.

3. Composition cosmétique selon l'une quelconque des revendications précédentes, **caractérisée en ce que** le rapport molaire des monomères (i) sur les monomères (ii) est compris entre 5 : 1 et 0,5 : 1, particulièrement entre 4 : 1 et 1 : 1, de préférence entre 3 : 1 et 3 : 2.

4. Composition cosmétique selon l'une quelconque des revendications précédentes, **caractérisée en ce que** le rapport molaire des monomères (i) sur les monomères (iii) est compris entre 5 : 1 et 0,5 : 1, particulièrement entre 3 : 1 et 1 : 1, de préférence entre 2,5 : 1 et 1,1 : 1.

5. Composition cosmétique selon l'une quelconque des revendications précédentes, **caractérisée en ce que** le copolymère hyperramifié a un poids moléculaire moyen en nombre Mₙ compris entre 1 400 et 3 000 g/mole, de préférence entre 2 100 et 2 300 g/mole.

6. Composition cosmétique selon l'une quelconque des revendications précédentes, **caractérisée en ce que** le copolymère hyperramifié est le polyquaternium-110.

7. Composition cosmétique selon l'une quelconque des revendications précédentes, **caractérisée en ce que** la composition est exempte de polyvinylpyrrolidones (PVP).

8. Composition cosmétique selon l'une quelconque des revendications précédentes, **caractérisée en ce que** la composition cosmétique est une composition de soin de la peau.

9. Composition cosmétique selon l'une quelconque des revendications précédentes, **caractérisée en ce que** la composition cosmétique est une composition cosmétique sans sulfate.

10. Composition cosmétique selon l'une quelconque des revendications précédentes, **caractérisée en ce que** la composition cosmétique est exempte d'émulsifiant cationique.

11. Utilisation d'un copolymère hyperramifié des monomères
(i) anhydride d'acide dodécénylsuccinique
(ii) diisopropanolamine
(iii) bis-diméthylaminopropylamine
ayant des groupes terminaux de formule 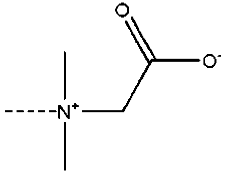
et ayant un poids moléculaire Mn compris entre 1 200 et 4 000 g/mole
dans une composition cosmétique comprenant au moins une huile ayant un indice de polarité soit compris dans la plage de 50-30 mN/m, soit dans la plage de 18-5 mN/m pour réduire le transfert de matière vers une surface en contact ;
tandis que l'indice de polarité de l'huile est la tension interfaciale de l'huile par rapport à l'eau et est déterminé à l'aide d'un tensiomètre à anneau par analogie avec le procédé de la norme ASTM D971-99a (2004).

12. Utilisation selon la revendication 11, **caractérisée en ce que** la surface en contact est une surface de verre.

13. Utilisation selon la revendication 11 ou 12, **caractérisée en ce que** la surface en contact est un affichage d'un téléphone mobile, d'un ordinateur ou d'une tablette.
